# EUROPEAN PATENT APPLICATION

(11) **EP 4 760 727 A1**
(43) Date of publication of application: **17.06.2026**
(21) Application number: 24871899.1
(22) Date of filing: 12.09.2024
(51) Int. Cl.: G16B 30/00, G16B 40/00

(54) **INFORMATION PROCESSING DEVICE, OPERATION METHOD FOR INFORMATION PROCESSING DEVICE, AND OPERATION PROGRAM FOR INFORMATION PROCESSING DEVICE**

(30) Priority: 28.09.2023 JP 2023167582
(71) Applicant: FUJIFILM Corporation, Tokyo 106-8620 (JP)
(72) Inventor: TSUTAOKA, Takuya, Ashigarakami-gun, Kanagawa 258-8577 (JP)
(74) Representative: Hoffmann Eitle
(86) International application number: PCT/JP2024/032771
(87) International publication number: WO 2025/070104

(57) **Abstract**

An information processing apparatus includes: a processor, in which the processor is configured to: acquire structural information of an amino acid sequence unique to a protein and a feature amount of an amino acid residue constituting the amino acid sequence; set a weight of the feature amount based on the structural information; and cause a machine learning model to predict property information indicating a property of the protein by applying the feature amount to which the weight is set to the machine learning model.

## Description

### BACKGROUND OF THE INVENTION

### 1. Field of the Invention

The technology of the present disclosure relates to an information processing apparatus, an operation method of an information processing apparatus, and an operation program of an information processing apparatus.

Recently, pharmaceuticals such as biopharmaceuticals, peptide pharmaceuticals, and nucleic acid pharmaceuticals have attracted attention due to high drug efficacy and low side effects. For example, the biopharmaceutical has a protein such as interferon or an antibody as an active ingredient. For example, the protein such as an antibody has property information indicating a property, such as a hydrogen ion exponent (potential of hydrogen (pH) value) at which the highest activity is obtained or the quality is most stably maintained, which is different for each type.

JP2021-158973A discloses a technique of predicting thermal stability (whether or not a heat resistance temperature is higher than a set temperature) of a protein variant having an amino acid substitution mutation in an amino acid sequence by using a machine learning model. In JP2021-158973A, in order to improve prediction accuracy of thermal stability, a feature amount (type and number of amino acid residues in a local region, size of amino acid residues in a local region, angle of a side chain, hydrophobicity, and the like) of an amino acid residue in a local region of a protein variant centered on a portion of an amino acid substitution mutation is selectively applied to a machine learning model.

### SUMMARY OF THE INVENTION

The technique disclosed in JP2021-158973A targets a protein variant. Therefore, the technique cannot be used for a general protein that is not a variant.

One embodiment according to the technology of the present disclosure provides an information processing apparatus, an operation method of an information processing apparatus, and an operation program of an information processing apparatus that can predict property information indicating a property of a protein with high accuracy.

An information processing apparatus according to the present disclosure includes a processor, in which the processor is configured to: acquire structural information of an amino acid sequence unique to a protein and a feature amount of an amino acid residue constituting the amino acid sequence; set a weight of the feature amount based on the structural information; and cause a machine learning model to predict property information indicating a property of the protein by applying the feature amount to which the weight is set to the machine learning model.

It is preferable that the structural information is partition region information indicating a functional partition region of the amino acid residue.

It is preferable that the protein is an antibody, the partition region includes a complementarity determining region, and the processor is configured to set a weight of a feature amount of the amino acid residue in which the partition region indicated by the partition region information is the complementarity determining region to be higher than a weight of a feature amount of the amino acid residue in which the partition region indicated by the partition region information is not the complementarity determining region.

It is preferable that the structural information is three-dimensional structural information of the amino acid sequence.

It is preferable that the three-dimensional structural information is three-dimensional shape information indicating a three-dimensional shape of the amino acid sequence.

It is preferable that the three-dimensional shape includes a loop shape and a linear shape, and the processor is configured to set a weight of a feature amount of the amino acid residue in which the three-dimensional shape indicated by the three-dimensional shape information is the loop shape to be higher than a weight of a feature amount of the amino acid residue in which the three-dimensional shape indicated by the three-dimensional shape information is the linear shape.

It is preferable that the processor is configured to perform preprocessing according to the weight on the feature amount of the amino acid residue or the machine learning model.

It is preferable that the preprocessing is processing for calculating a weighted average of the feature amounts of the amino acid residues according to the weight.

It is preferable that the preprocessing is processing for setting an initial coefficient according to the weight in the machine learning model.

It is preferable that the property information is an optimal hydrogen ion exponent of the protein.

It is preferable that the protein is an antibody.

An operation method of an information processing apparatus according to the present disclosure includes acquiring structural information of an amino acid sequence unique to a protein and a feature amount of an amino acid residue constituting the amino acid sequence, setting a weight of the feature amount based on the structural information, and causing a machine learning model to predict property information indicating a property of the protein by applying the feature amount to which the weight is set to the machine learning model.

An operation program of an information processing apparatus according to the present disclosure causes a computer to execute a process including acquiring structural information of an amino acid sequence unique to a protein and a feature amount of an amino acid residue constituting the amino acid sequence, setting a weight of the feature amount based on the structural information, and causing a machine learning model to predict property information indicating a property of the protein by applying the feature amount to which the weight is set to the machine learning model.

According to the technology of the present disclosure, it is possible to provide an information processing apparatus, an operation method of an information processing apparatus, and an operation program of an information processing apparatus that can predict property information indicating a property of a protein with high accuracy.

### BRIEF DESCRIPTION OF THE DRAWINGS

FIG. 1 is a diagram showing an information processing server and a user terminal.
FIG. 2 is a diagram showing a basic structure of an antibody.
FIG. 3 is a block diagram showing a computer constituting the information processing server and the user terminal.
FIG. 4 is a block diagram showing a processing unit of a CPU of the information processing server.
FIG. 5 is a diagram showing structure information.
FIG. 6 is a diagram showing processing for a feature amount extraction unit and a feature amount group.
FIG. 7 is a diagram showing setting auxiliary information.
FIG. 8 is a diagram showing processing for a weight setting unit.
FIG. 9 is a diagram showing a weighted feature amount group.
FIG. 10 is a diagram showing an aggregated feature amount.
FIG. 11 is a diagram showing processing for a prediction unit.
FIG. 12 is a diagram showing a neural network constituting a property information prediction model.
FIG. 13 is a block diagram showing a processing unit of a CPU of the user terminal.
FIG. 14 is a diagram showing an information input screen.
FIG. 15 is a diagram showing a prediction result display screen.
FIG. 16 is a flowchart showing a processing procedure of the information processing server.
FIG. 17 is a diagram showing another example of the structural information.
FIG. 18 is a diagram showing another example of the setting auxiliary information.
FIG. 19 is a diagram showing still another example of the structural information.
FIG. 20 is a diagram showing still another example of the setting auxiliary information.
FIG. 21 is a diagram showing processing for a preprocessing unit of a second embodiment.

### DESCRIPTION OF THE PREFERRED EMBODIMENTS

### [First Embodiment]

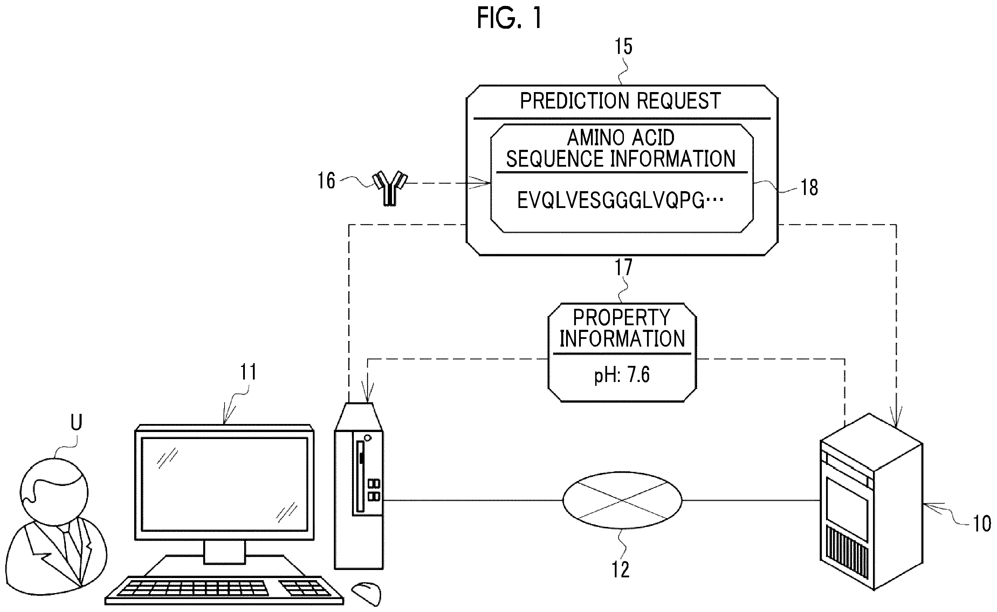

As shown in FIG. 1 as an example, an information processing server 10 is connected to a user terminal 11 via a network 12. The information processing server 10 is an example of an "information processing apparatus" according to the technology of the present disclosure.

The user terminal 11 is installed in a pharmaceutical company that develops biopharmaceuticals, or in an organization that undertakes development work of biopharmaceuticals from a pharmaceutical company, that is, a contract research organization (CRO). The user terminal 11 is operated by a user U who is involved in the development of the biopharmaceuticals in the pharmaceutical company or the contract research organization (hereinafter, collectively referred to as a pharmaceutical facility). The network 12 is, for example, a wide area network (WAN) such as the Internet or a public communication network. In FIG. 1, only one user terminal 11 is connected to the information processing server 10, but in reality, a plurality of user terminals 11 of a plurality of pharmaceutical facilities are connected to the information processing server 10.

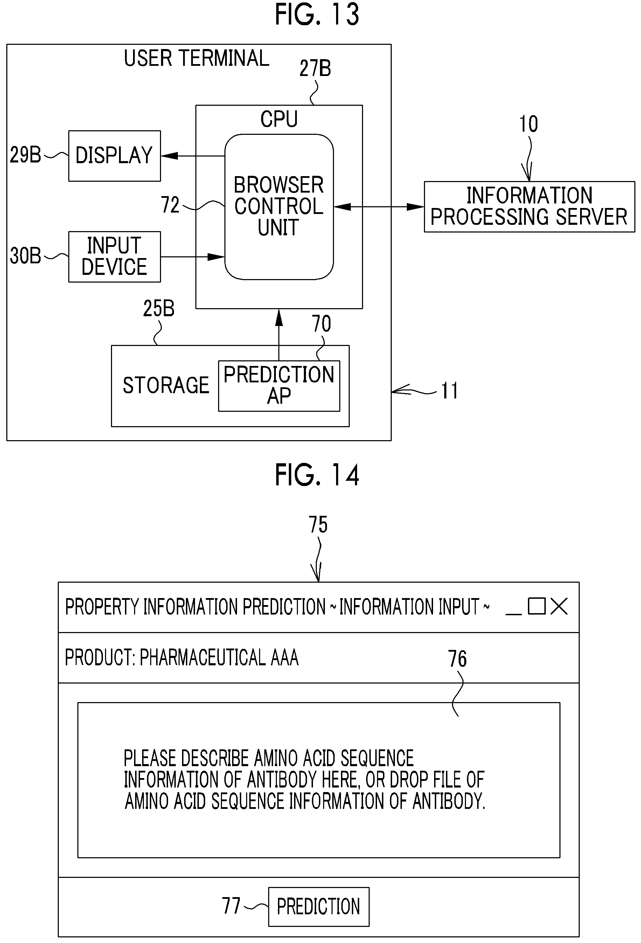

The user terminal 11 transmits a prediction request 15 to the information processing server 10. The prediction request 15 is a request for the information processing server 10 to predict property information 17 indicating a property of an antibody 16 that is an active ingredient of a biopharmaceutical. The prediction request 15 includes amino acid sequence information 18 of the antibody 16. The amino acid sequence information 18 is identified by an experiment and is input by the user U operating an input device 30B (see FIG. 13) of the user terminal 11. The antibody 16 is an example of a "protein" according to the technology of the present disclosure. Although not shown, the prediction request 15 also includes a terminal identification data (ID) or the like for uniquely identifying the user terminal 11 that is a transmission source of the prediction request 15.

The amino acid sequence information 18 describes an order of peptide bonds of the amino acid residues constituting the antibody 16 from an amino terminal toward a carboxyl terminal using one-letter alphabet abbreviations representing the amino acid residue. Since there are about 450 amino acid residues constituting the antibody 16, the alphabet of the amino acid sequence information 18 is also about 450. For example, the abbreviation "E" is glutamic acid, "L" is leucine, and "G" is glycine. Such an amino acid residue sequence is also referred to as a primary structure.

In a case where the prediction request 15 is received, the information processing server 10 predicts the property information 17 based on the amino acid sequence information 18. Here, the property information 17 is a hydrogen ion exponent of a storage solution of the antibody 16 in which the quality of the antibody 16 is most stably maintained, that is, an optimal hydrogen ion exponent of the antibody 16. The information processing server 10 delivers the property information 17 to the user terminal 11 that is the transmission source of the prediction request 15. In a case where the user terminal 11 receives the property information 17, the user terminal 11 displays the property information 17 on a display 29B (see FIG. 13) of the user terminal 11 and provides the property information 17 for viewing by the user U.

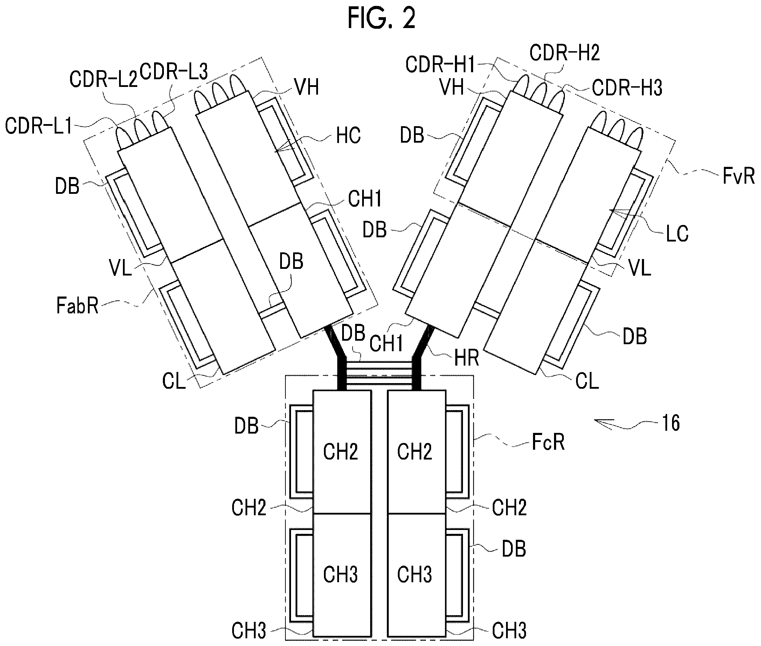

As shown in FIG. 2 as an example, the antibody 16 basically has four polypeptide chains, that is, two identical heavy chains HC and two identical light chains LC. The antibody 16 has a configuration in which the two heavy chains HC and the two light chains LC are bonded by a disulfide bond DB, and has a Y shape that is bilaterally symmetrical.

The heavy chain HC is composed of a variable domain VH (variable domain, heavy chain) and constant domains CH (constant domain, heavy chain) 1, CH2, and CH3. The light chain LC is composed of a variable domain VL (variable domain, light chain) and a constant domain CL (constant domain, light chain). The constant domains CH1 and CH2 of the heavy chain HC are connected by a hinge region HR. The variable domains VH and VL are collectively referred to as a variable region. The constant domains CH1 to CH3 and CL are collectively referred to as a constant region. The variable domains VH and VL, and the constant domains CH1 to CH3 and CL are examples of a "partition region" according to the technology of the present disclosure.

The variable domain VH includes three complementarity determining regions CDR-H (complementarity determining region, heavy chain) 1, CDR-H2, and CDR-H3. In addition, the variable domain VL also includes three complementarity determining regions CDR-L (complementarity determining region, light chain) 1, CDR-L2, and CDR-L3. These complementarity determining regions CDR-H1 to CDR-H3 and CDR-L1 to CDR-L3 are bonding sites with an antigen and are also referred to as a hypervariable region. The complementarity determining regions CDR-H1 to CDR-H3 and CDR-L1 to CDR-L3 are also examples of a "partition region" according to the technology of the present disclosure.

A region composed of the variable domains VH and VL and the constant domains CH1 and CL is the fragment antigen-binding region FabR. A region composed of the constant domains CH2 and CH3 and a part of the hinge region HR is the fragment crystallizable region FcR. A region composed of the variable domains VH and VL is the fragment variable region FvR. As is well known, the fragment antigen-binding region FabR and the fragment crystallizable region FcR can be produced by papain digestion. Similarly, the fragment variable region FvR can be produced by pepsin digestion.

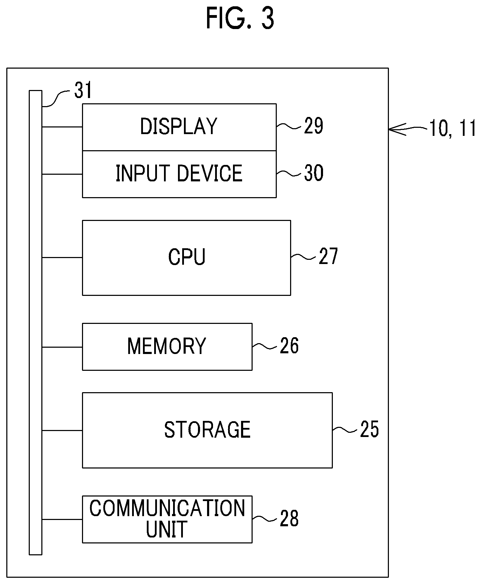

As shown in FIG. 3 as an example, computers constituting the information processing server 10 and the user terminal 11 basically have the same configuration, and comprise a storage 25, a memory 26, a central processing unit (CPU) 27, a communication unit 28, a display 29, and an input device 30. These are connected to each other through a bus line 31.

The storage 25 is a hard disk drive that is built in the computers constituting the information processing server 10 and the user terminal 11 or connected thereto through a cable or a network. Alternatively, the storage 25 is a disk array obtained by connecting a plurality of hard disk drives. The storage 25 stores a control program such as an operating system, various application programs (hereinafter, referred to as an application program (AP)), various types of data associated with these programs, and the like. A solid state drive may be used instead of the hard disk drive.

The memory 26 is a work memory for executing processing via the CPU 27. The CPU 27 loads the programs stored in the storage 25 into the memory 26 and executes processing in accordance with the programs. Accordingly, the CPU 27 controls each unit of the computer in an integrated manner. The CPU 27 is an example of a "processor" according to the technology of the present disclosure. The memory 26 may be incorporated into the CPU 27.

The communication unit 28 controls transmission of various information to an external apparatus. The display 29 displays various screens. The various screens have an operation function by a graphical user interface (GUI). The computers constituting the information processing server 10 and the user terminal 11 receive input of an operation instruction from the input device 30 through various screens. The input device 30 is a keyboard, a mouse, a touch panel, a microphone for audio input, or the like.

Further, in the following description, the subscript "A" is attached to the reference numerals indicating each unit (the storage 25 and the CPU 27) of the computer constituting the information processing server 10, and the subscript "B" is attached to the reference numerals indicating each unit (the storage 25, the CPU 27, the display 29, and the input device 30) of the computer constituting the user terminal 11 to distinguish the units.

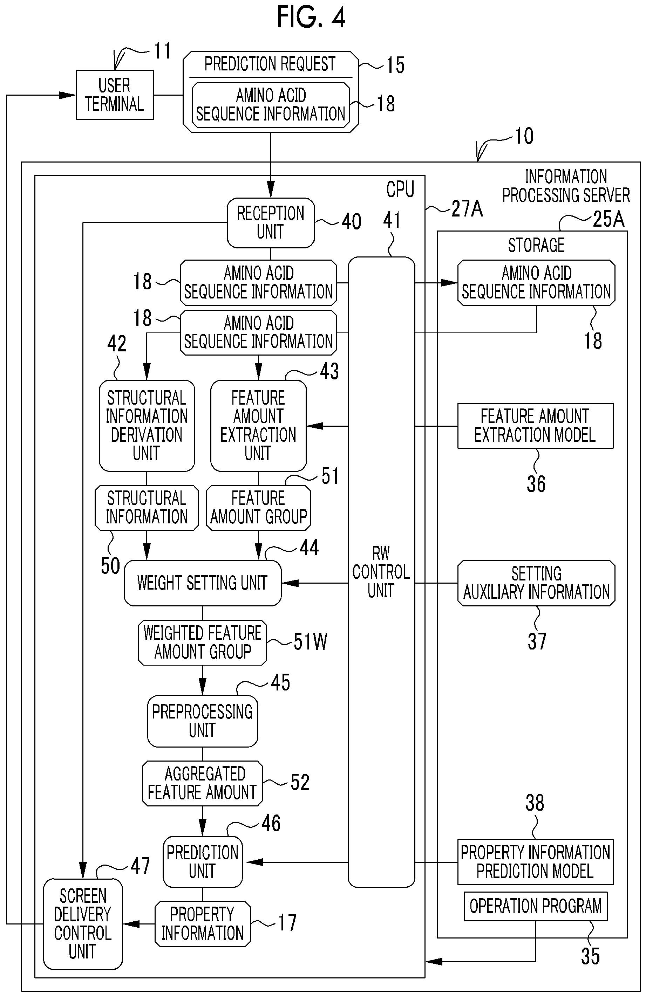

As shown in FIG. 4 as an example, an operation program 35 is stored in a storage 25A of the information processing server 10. The operation program 35 is an AP for causing the computer to function as the information processing server 10. That is, the operation program 35 is an example of an "operation program of an information processing apparatus" according to the technology of the present disclosure. The storage 25A also stores a feature amount extraction model 36, setting auxiliary information 37, and a property information prediction model 38. The property information prediction model 38 is an example of a "machine learning model" according to the disclosed technology.

In a case where the operation program 35 is started, the CPU 27 of the computer constituting the information processing server 10 functions as a reception unit 40, a read and write (hereinafter, abbreviated as RW) control unit 41, a structural information derivation unit 42, a feature amount extraction unit 43, a weight setting unit 44, a preprocessing unit 45, a prediction unit 46, and a screen delivery control unit 47 in cooperation with the memory 26 and the like.

The reception unit 40 receives various requests from the user terminal 11. In particular, the reception unit 40 receives the prediction request 15 from the user terminal 11. As described above, the prediction request 15 includes the amino acid sequence information 18. Therefore, the reception unit 40 acquires the amino acid sequence information 18 by receiving the prediction request 15. The reception unit 40 outputs the amino acid sequence information 18 to the RW control unit 41. In addition, the reception unit 40 outputs a terminal ID of the user terminal 11 (not shown) to the screen delivery control unit 47.

The RW control unit 41 controls storage of various types of data in the storage 25A and readout of various types of data from the storage 25A. For example, the RW control unit 41 stores the amino acid sequence information 18 from the reception unit 40 in the storage 25A. In addition, the RW control unit 41 reads out the amino acid sequence information 18 from the storage 25A and outputs the readout amino acid sequence information 18 to the structural information derivation unit 42 and the feature amount extraction unit 43.

The RW control unit 41 reads out the feature amount extraction model 36 from the storage 25A and outputs the readout feature amount extraction model 36 to the feature amount extraction unit 43. In addition, the RW control unit 41 reads out the setting auxiliary information 37 from the storage 25A and outputs the readout setting auxiliary information 37 to the weight setting unit 44. Further, the RW control unit 41 reads out the property information prediction model 38 from the storage 25A and outputs the readout property information prediction model 38 to the prediction unit 46.

The structural information derivation unit 42 derives structural information 50 of an amino acid sequence unique to the antibody 16 based on the amino acid sequence information 18. The structural information derivation unit 42 outputs the structural information 50 to the weight setting unit 44.

The feature amount extraction unit 43 extracts a feature amount group 51 that is a set of feature amounts of each amino acid residue constituting the amino acid sequence of the antibody 16 by applying the amino acid sequence information 18 to the feature amount extraction model 36. The feature amount extraction unit 43 outputs the feature amount group 51 to the weight setting unit 44.

The weight setting unit 44 sets the weight of each feature amount constituting the feature amount group 51 based on the structural information 50 while referring to the setting auxiliary information 37. The weight setting unit 44 registers the set weights in the feature amount group 51, thereby obtaining a weighted feature amount group 51W. The weight setting unit 44 outputs the weighted feature amount group 51W to the preprocessing unit 45.

The preprocessing unit 45 performs preprocessing according to the weights on the feature amounts registered in the weighted feature amount group 51W to generate an aggregated feature amount 52. The preprocessing unit 45 outputs the aggregated feature amount 52 to the prediction unit 46.

The prediction unit 46 causes the property information prediction model 38 to predict the property information 17 by applying the aggregated feature amount 52 to the property information prediction model 38. The prediction unit 46 outputs the property information 17 to the screen delivery control unit 47.

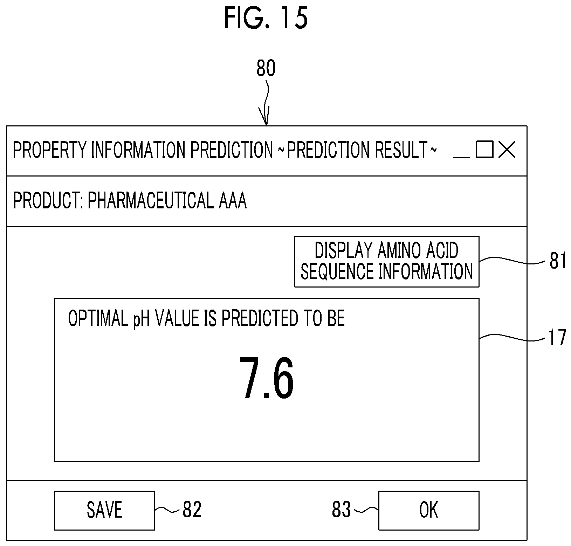

The screen delivery control unit 47 performs control of delivering various screens to the user terminal 11. Specifically, the screen delivery control unit 47 delivers output of the various screens to the user terminal 11 that is a transmission source of the various requests, in the form of screen data for web delivery created using a markup language such as extensible markup language (XML). In this case, the screen delivery control unit 47 specifies the user terminal 11 that is the transmission source of various requests based on the terminal ID from the reception unit 40. The various screens include an information input screen 75 (see FIG. 14) for inputting the amino acid sequence information 18, a prediction result display screen 80 (see FIG. 15) for displaying the property information 17, and the like. Note that, instead of XML, another data description language, such as JavaScript (registered trademark) Object Notation (JSON), may be used.

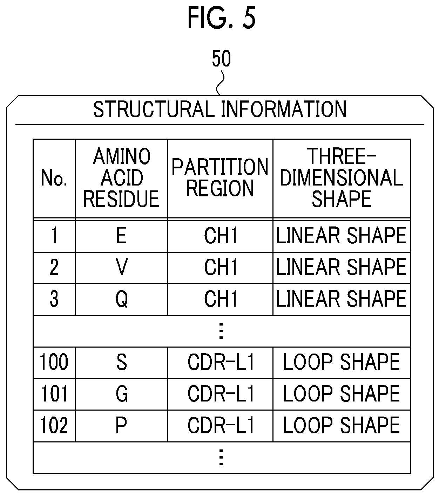

As shown in FIG. 5 as an example, the structural information 50 is information in which a functional partition region of the amino acid residue and a three-dimensional shape (hereinafter, referred to as a three-dimensional shape) of a three-dimensional structure formed by the amino acid sequence are registered for each amino acid residue constituting the amino acid sequence of the antibody 16. That is, the structural information 50 is an example of "partition region information" and "three-dimensional shape information" according to the disclosed technology, and is also an example of "three-dimensional structural information".

Any of the variable domains VH and VL, the constant domains CH1 to CH3 and CL, or the complementarity determining regions CDR-H1 to CDR-H3 and CDR-L1 to CDR-L3 shown in FIG. 2 is registered in the partition region. Any of a linear shape or a loop shape is registered in the three-dimensional shape.

The structural information derivation unit 42 specifies, for example, the partition region of the antibody 16 whose partition region is unknown by performing multiple alignment with a set of antibodies 16 whose partition regions are known. The partition region can be obtained not only by analyzing the antibody 16 by a well-known technique such as X-ray crystal structure analysis, but also from paper information or a database such as Protein Data Bank.

In addition, the structural information derivation unit 42, for example, inputs the amino acid sequence information 18 to "AlphaFold2" that is a program for predicting a three-dimensional structure of a protein developed by DeepMind Technologies Limited. Then, three-dimensional coordinates of each amino acid residue are output from "AlphaFold2", and the three-dimensional shape is specified based on the three-dimensional coordinates.

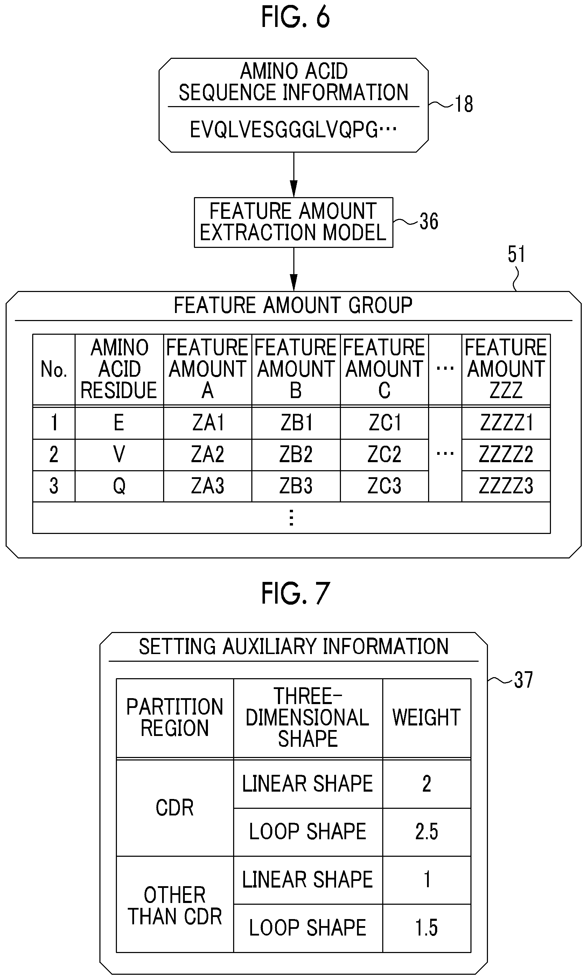

As shown in FIG. 6 as an example, the feature amount extraction unit 43 inputs the amino acid sequence information 18 to the feature amount extraction model 36 to output the feature amount group 51 from the feature amount extraction model 36. The feature amount extraction model 36 is, for example, an encoder unit of a neural network or a machine learning model such as a support vector machine (SVM). The feature amount extraction model 36 may be trained in the information processing server 10 or in a device other than the information processing server 10. In addition, the feature amount extraction model 36 may be continuously trained even after the operation.

There are a plurality of types of feature amounts, such as a feature amount A, a feature amount B, a feature amount C, ..., and a feature amount ZZZ. A plurality of types of feature amounts are registered in each amino acid residue constituting the amino acid sequence of the antibody 16. For example, for the amino acid residue "E" at No. 1, ZA1 is registered as the feature amount A, ZB1 is registered as the feature amount B, ZC1 is registered as the feature amount C, ..., and ZZZZ1 is registered as the feature amount ZZZ, respectively. As described above, each amino acid residue can be represented by a plurality of types of feature amounts. The plurality of types of feature amounts are multidimensional vector data. The number of types of feature amounts (the number of dimensions of the feature amount vector) is several hundred to several thousand. In addition to or instead of the machine learning model such as the feature amount extraction model 36, the feature amount may be extracted by a molecular dynamics (MD) method.

As shown in FIG. 7 as an example, the setting auxiliary information 37 is information in which the weight to be set is registered for each combination of the partition region and the three-dimensional shape. The partition region is divided into two groups of a complementarity determining region CDR and a region other than the complementarity determining region CDR. The two groups are further divided into whether the three-dimensional shape is a linear shape or a loop shape. In a case where the partition region is the complementarity determining region CDR, the weight in a case where the three-dimensional shape is the linear shape is 2, and the weight in a case where the three-dimensional shape is the loop shape is 2.5. In a case where the partition region is a region other than the complementarity determining region CDR, the weight in a case where the three-dimensional shape is the linear shape is 1, and the weight in a case where the three-dimensional shape is the loop shape is 1.5. In a case where the partition region is the complementarity determining region CDR, the weight is set to be higher than in a case where the partition region is a region other than the complementarity determining region CDR. In addition, in a case where the three-dimensional shape is the loop shape, the weight is set to be higher than in a case where the three-dimensional shape is the linear shape.

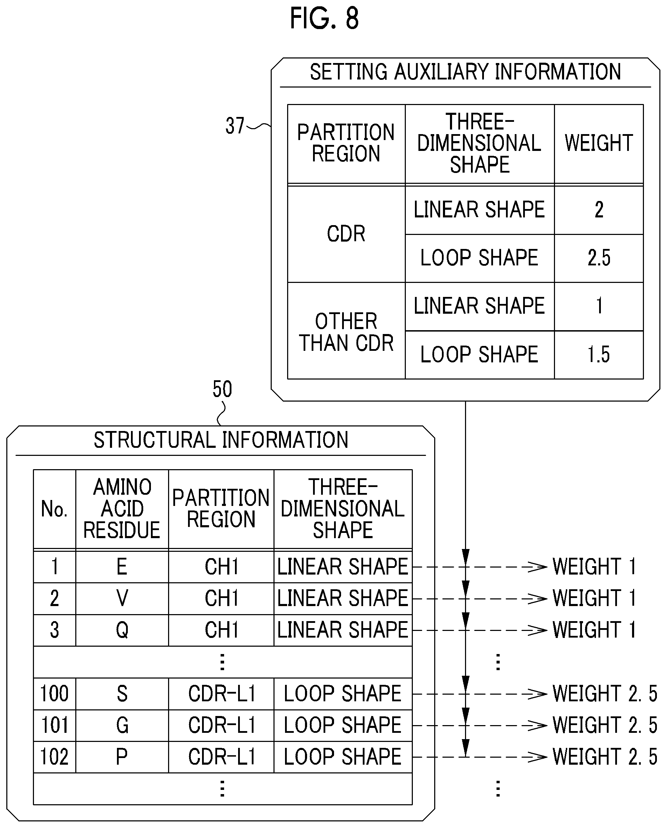

As shown in FIG. 8 as an example, the weight setting unit 44 sets the weight corresponding to the combination of the partition region and the three-dimensional shape of the structural information 50 to each amino acid residue by referring to the setting auxiliary information 37. More specifically, the weight setting unit 44 sets the weight of the feature amount of the amino acid residue in which the partition region is the complementarity determining region CDR to be higher than the weight of the feature amount of the amino acid residue in which the partition region is not the complementarity determining region CDR. In addition, the weight setting unit 44 sets the weight of the feature amount of the amino acid residue in which the three-dimensional shape is the loop shape to be higher than the weight of the feature amount of the amino acid residue in which the three-dimensional shape is the linear shape. For example, for the amino acid residue "Q" at No. 3, since the partition region is the constant domain CH1 and the three-dimensional shape is the linear shape, the weight setting unit 44 sets a weight of 1. In addition, for example, for the amino acid residue "S" at No. 100, since the partition region is the complementarity determining region CDR-L1 and the three-dimensional shape is the loop shape, the weight setting unit 44 sets a weight of 2.5.

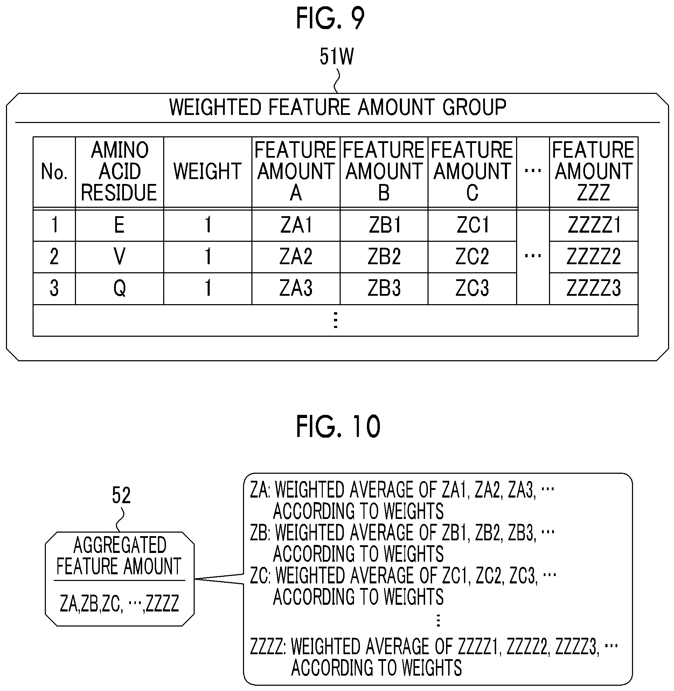

As shown in FIG. 9 as an example, the weighted feature amount group 51W is obtained by adding a weight term to the feature amount group 51.

As shown in FIG. 10 as an example, the preprocessing unit 45 calculates a weighted average according to the weights of the respective feature amounts as preprocessing before applying the feature amounts to the property information prediction model 38, thereby obtaining the aggregated feature amount 52. Specifically, the preprocessing unit 45 calculates a weighted average ZA of ZA1, ZA2, ZA3, ... of the feature amount A according to the weights, a weighted average ZB of ZB1, ZB2, ZB3, ... of the feature amount B according to the weights, a weighted average ZC of ZC1, ZC2, ZC3, ... of the feature amount C according to the weights, ..., and a weighted average ZZZZ of ZZZZ1, ZZZZ2, ZZZZ3, ... of the feature amount ZZZ according to the weights, respectively. Then, a set of the weighted averages ZA, ZB, ZC, ..., and ZZZZ is output as the aggregated feature amount 52.

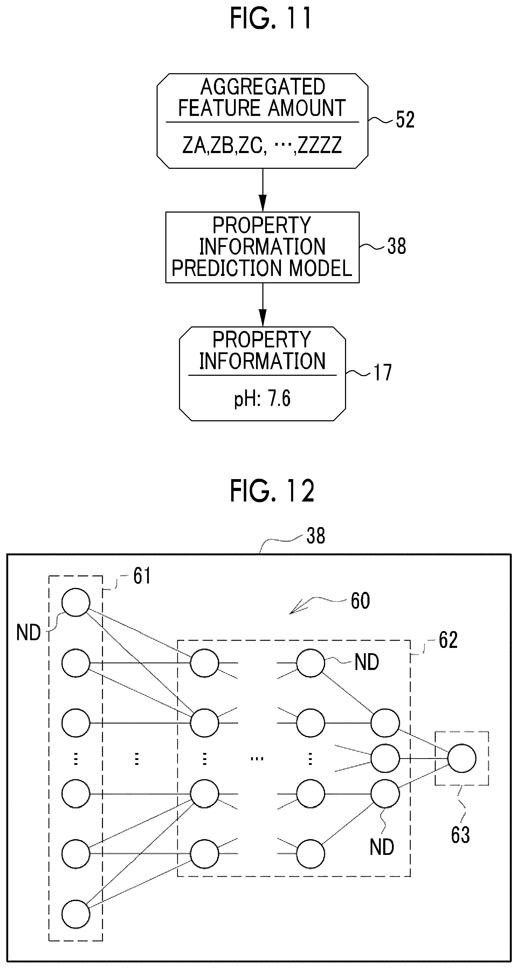

As shown in FIG. 11 as an example, the prediction unit 46 inputs the aggregated feature amount 52 to the property information prediction model 38 and causes the property information prediction model 38 to output the property information 17.

As shown in FIG. 12 as an example, the property information prediction model 38 is constructed by a neural network 60. As is well known, the neural network 60 has an input layer 61, an interlayer (also referred to as a hidden layer) 62, and an output layer 63. The input layer 61, the interlayer 62, and the output layer 63 each have a plurality of nodes ND. A coefficient indicating the connection strength between the respective nodes ND is set between the node ND of the input layer 61 and the node ND of the interlayer 62, between the nodes ND of the interlayer 62, and between the node ND of the interlayer 62 and the node ND of the output layer 63. A suitable activation function, such as a linear function or a rectified linear unit (ReLU) function, is set for the node ND of the output layer 63.

Each feature amount ZA, ZB, ZC, ..., and ZZZZ of the aggregated feature amount 52 is input to each node ND of the input layer 61. In addition, the property information 17 is output from the node ND of the output layer 63. In addition, the property information prediction model 38 is not limited to the illustrated neural network 60, and may be another machine learning model such as a decision tree, a gradient boosting decision tree, a random forest, a support vector machine, and a naive Bayes model.

The property information prediction model 38 may be trained in the information processing server 10 or in a device other than the information processing server 10. In addition, the property information prediction model 38 may be continuously trained even after the operation.

As shown in FIG. 13 as an example, a prediction AP 70 is stored in the storage 25B of the user terminal 11. The prediction AP 70 is installed in the user terminal 11 by the user U. The prediction AP 70 is an AP for predicting the property information 17 of the antibody 16. In a case where the prediction AP 70 is activated, a CPU 27B of the user terminal 11 functions as a browser control unit 72 in cooperation with the memory 26 and the like. The browser control unit 72 controls an operation of a dedicated web browser of the prediction AP 70.

The browser control unit 72 reproduces various screens based on various types of screen data from the information processing server 10 and displays the reproduced various screens on the display 29B. Additionally, the browser control unit 72 receives various operation instructions input by the user U from the input device 30B through various screens. The browser control unit 72 transmits various requests corresponding to the operation instructions including the prediction request 15 to the information processing server 10.

In a case where the prediction AP 70 is activated, the information input screen 75 shown in FIG. 14 as an example is displayed on the display 29B under the control of the browser control unit 72. The information input screen 75 is provided with an input box 76 for the amino acid sequence information 18. In the input box 76, the amino acid sequence information 18 can be described or a file of the amino acid sequence information 18 can be dropped.

The user U inputs desired amino acid sequence information 18 into the input box 76 and then selects a prediction button 77. In a case where the prediction button 77 is selected, the browser control unit 72 generates the prediction request 15 including the amino acid sequence information 18 input to the input box 76, and transmits the generated prediction request 15 to the information processing server 10.

In addition, in a case where the prediction of the property information 17 is performed in the information processing server 10, the prediction result display screen 80 shown in FIG. 15 as an example is displayed on the display 29B under the control of the browser control unit 72. The property information 17, that is, a message representing the property information 17 is displayed on the prediction result display screen 80. As described above, the property information 17 is presented to the user U in a form of delivery of screen data.

An amino acid sequence information display button 81 is provided at an upper part of the prediction result display screen 80. In a case where the amino acid sequence information display button 81 is selected, a display screen of the amino acid sequence information 18 is displayed in a pop-up manner.

In addition, a save button 82 and an OK button 83 are provided at a lower part of the prediction result display screen 80. In a case where the save button 82 is selected, the property information 17 and the amino acid sequence information 18 are stored in association with each other in the storage 25B of the user terminal 11. In a case where the OK button 83 is selected, the display of the prediction result display screen 80 is cleared.

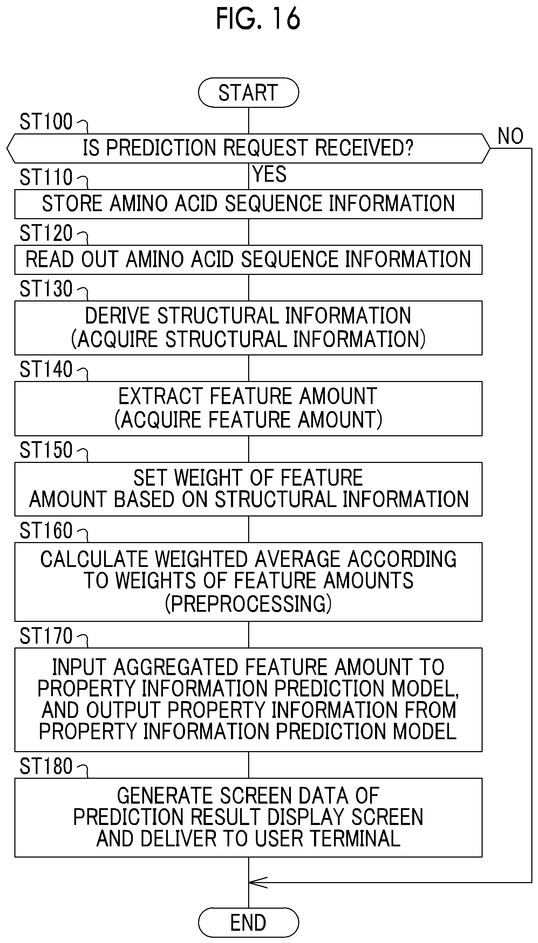

Next, an operation according to the configuration will be described with reference to a flowchart in FIG. 16. First, in a case where the operation program 35 is started in the information processing server 10, as shown in FIG 4, the CPU 27A of the information processing server 10 functions as the reception unit 40, the RW control unit 41, the structural information derivation unit 42, the feature amount extraction unit 43, the weight setting unit 44, the preprocessing unit 45, the prediction unit 46, and the screen delivery control unit 47. In addition, in a case where the prediction AP 70 is activated in the user terminal 11, as shown in FIG. 13, the CPU 27B of the user terminal 11 functions as the browser control unit 72.

The information input screen 75 shown in FIG. 14 is displayed on the display 29B of the user terminal 11 under the control of the browser control unit 72. In a case where the user U inputs the desired amino acid sequence information 18 into the input box 76 and selects the prediction button 77 on the information input screen 75, the prediction request 15 is transmitted from the browser control unit 72 to the information processing server 10. As shown in FIG. 1, the prediction request 15 includes the amino acid sequence information 18 and the terminal ID of the user terminal 11.

In the information processing server 10, the prediction request 15 is received by the reception unit 40 (YES in step ST100). The amino acid sequence information 18 of the prediction request 15 is output from the reception unit 40 to the RW control unit 41 and is stored in the storage 25A under the control of the RW control unit 41 (step ST110).

The RW control unit 41 reads out the amino acid sequence information 18 from the storage 25A (step ST120). The amino acid sequence information 18 is output from the RW control unit 41 to the structural information derivation unit 42 and the feature amount extraction unit 43.

In the structural information derivation unit 42, the structural information 50 shown in FIG. 5 is derived based on the amino acid sequence information 18 (step ST130). The structural information 50 is output from the structural information derivation unit 42 to the weight setting unit 44.

In addition, as shown in FIG. 6, in the feature amount extraction unit 43, the amino acid sequence information 18 is input to the feature amount extraction model 36, and the feature amount group 51 is output from the feature amount extraction model 36 (step ST140). The feature amount group 51 is output from the feature amount extraction unit 43 to the weight setting unit 44.

Next, as shown in FIG. 8, in the weight setting unit 44, the weight of the feature amount is set based on the structural information 50 while referring to the setting auxiliary information 37 (step ST150). In the weight setting unit 44, the weighted feature amount group 51W including the set weight term is generated. The weighted feature amount group 51W is output from the weight setting unit 44 to the preprocessing unit 45.

As shown in FIG. 10, in the preprocessing unit 45, the weighted average according to the weights of the respective feature amounts is calculated as the preprocessing, and the aggregated feature amount 52 is generated (step ST160). The aggregated feature amount 52 is output from the preprocessing unit 45 to the prediction unit 46.

As shown in FIG. 11, in the prediction unit 46, the aggregated feature amount 52 is input to the property information prediction model 38, and the property information 17 is output from the property information prediction model 38 (step ST170). The property information 17 is output from the prediction unit 46 to the screen delivery control unit 47.

The screen delivery control unit 47 generates screen data of the prediction result display screen 80 including the message representing the property information 17. The screen data of the prediction result display screen 80 is delivered to the user terminal 11 that is the transmission source of the prediction request 15 under the control of the screen delivery control unit 47 (step ST180).

The prediction result display screen 80 shown in FIG. 15 is displayed on the display 29B of the user terminal 11. The user U views the prediction result display screen 80 and checks the optimal hydrogen ion exponent of the property information 17.

As described above, the CPU 27A of the information processing server 10 comprises the structural information derivation unit 42, the feature amount extraction unit 43, the weight setting unit 44, and the prediction unit 46. The structural information derivation unit 42 acquires the structural information 50 of the amino acid sequence unique to the antibody 16 by deriving the structural information 50. The feature amount extraction unit 43 acquires the feature amount of the amino acid residue constituting the amino acid sequence of the antibody 16 by extracting the feature amount. The weight setting unit 44 sets the weight of the feature amount based on the structural information 50. The prediction unit 46 causes the property information prediction model 38 to predict the property information 17 indicating the property of the antibody 16 by applying the feature amount to which the weight is set to the property information prediction model 38.

By setting the weight of the feature amount based on the structural information 50 of the antibody 16, the feature amount of the important amino acid residue that is considered to be deeply involved in the property in the structure of the antibody 16 can be used for the prediction of the property information 17. Therefore, it is possible to predict the property information 17 indicating the property of the antibody 16 with high accuracy.

The structural information 50 is information that can be derived regardless of whether or not the antibody 16 is a variant. Therefore, the technology of the present disclosure is not limited to being used only for a variant as in the technology disclosed in JP2021-158973A, and can be generally used for not only the variant but also a general antibody 16 that is not a variant.

As shown in FIG. 5, the structural information 50 is partition region information indicating a functional partition region of the amino acid residue. Then, the protein is the antibody 16, and the partition region includes the complementarity determining region CDR. As shown in FIGS. 7 and 8, the weight setting unit 44 sets the weight of the feature amount of the amino acid residue in which the partition region is the complementarity determining region CDR to be higher than the weight of the feature amount of the amino acid residue in which the partition region is not the complementarity determining region CDR.

As described above, the complementarity determining region CDR is a binding site to an antigen and is a region that determines the function of the antibody 16. In addition, the complementarity determining region CDR is generally exposed to a solvent such as a storage solution of the antibody 16. Therefore, in a case where the structural information 50 is set as the partition region information indicating the partition region including the complementarity determining region CDR, and the weight of the feature amount of the amino acid residue in which the partition region is the complementarity determining region CDR is set to be higher than the weight of the feature amount of the amino acid residue in which the partition region is not the complementarity determining region CDR, it is possible to predict the property information 17 with higher accuracy. In a case where the property information 17 is related to the storage solution of the antibody 16 such as the optimal hydrogen ion exponent of the antibody 16, the effect is particularly enhanced.

In addition, as shown in FIG. 5, the structural information 50 is three-dimensional structural information of the amino acid sequence, and the three-dimensional structural information is three-dimensional shape information indicating a three-dimensional shape of the amino acid sequence. Then, the three-dimensional shape includes a loop shape and a linear shape. As shown in FIGS. 7 and 8, the weight setting unit 44 sets the weight of the feature amount of the amino acid residue in which the three-dimensional shape is the loop shape to be higher than the weight of the feature amount of the amino acid residue in which the three-dimensional shape is the linear shape.

In a case where the three-dimensional shape is the loop shape, the amino acid residue is generally exposed to the solvent more than in a case where the three-dimensional shape is the linear shape. Therefore, in a case where the structural information 50 is set as the three-dimensional shape information indicating the three-dimensional shape of the amino acid sequence including the loop shape and the linear shape, and the weight of the feature amount of the amino acid residue in which the three-dimensional shape is the loop shape is set to be higher than the weight of the feature amount of the amino acid residue in which the three-dimensional shape is the linear shape, it is possible to predict the property information 17 with higher accuracy. In a case where the property information 17 is related to the storage solution of the antibody 16 such as the optimal hydrogen ion exponent of the antibody 16, the effect is particularly enhanced.

The preprocessing unit 45 performs preprocessing according to the weight on the feature amount of the amino acid residue. Specifically, as shown in FIG. 10, the preprocessing is processing for calculating a weighted average of the feature amounts of the amino acid residues according to the weights. Therefore, the feature amount in which the weight is reflected can be applied to the property information prediction model 38.

The optimal hydrogen ion exponent is very important for stably maintaining the quality of the antibody 16. Therefore, in a case where the property information 17 is set as the optimal hydrogen ion exponent of the antibody 16, it is possible to contribute to the stable maintenance of the quality of the antibody 16, and as a result, it is possible to sufficiently exhibit the drug efficacy of the biopharmaceutical having the antibody 16 as an active ingredient.

The biopharmaceutical containing the antibody 16 as the protein is called an antibody drug and is widely used not only for the treatment of chronic diseases, such as cancer, diabetes, and rheumatoid arthritis, but also for the treatment of rare diseases such as hemophilia and Crohn's disease. Therefore, according to the present example in which the protein is the antibody 16, it is possible to contribute to the stable supply of the antibody drugs widely used for the treatment of various diseases.

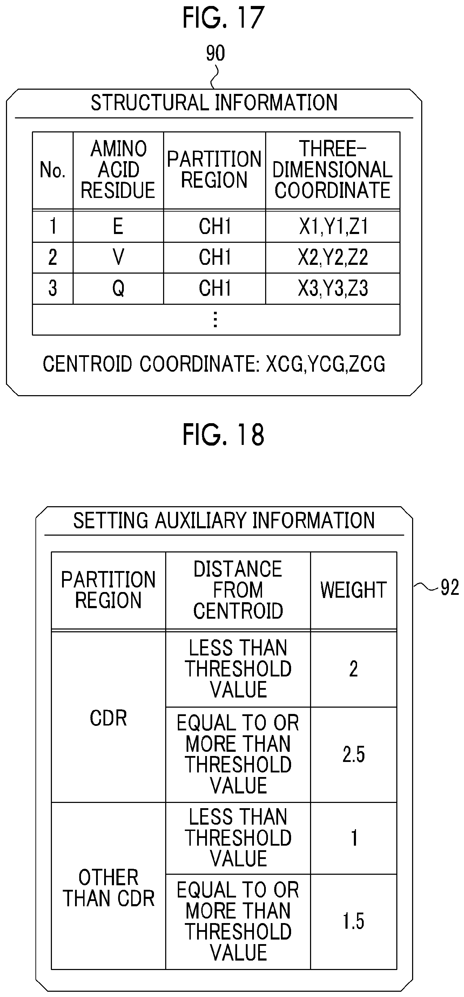

The three-dimensional structural information is not limited to the three-dimensional shape information given as an example. As the structural information 90 shown in FIG. 17 as an example, information on three-dimensional coordinates of each amino acid residue and centroid coordinates of the antibody 16 may be used. The three-dimensional coordinates of each amino acid residue and the centroid coordinates of the antibody 16 are obtained by inputting the amino acid sequence information 18 to "AlphaFold2".

In this case, the weight setting unit 44 sets the weight by referring to setting auxiliary information 92 shown in FIG. 18 as an example. The setting auxiliary information 92 has a term of a distance from the centroid instead of the term of the three-dimensional shape of the setting auxiliary information 37. The distance from the centroid can be calculated from the three-dimensional coordinates of each amino acid residue and the centroid coordinates of the antibody 16. The partition region is divided into two groups of the complementarity determining region CDR and a region other than the complementarity determining region CDR, as in the case of the setting auxiliary information 37. The two groups are further divided into whether the distance from the centroid is less than a threshold value or equal to or more than the threshold value. In a case where the partition region is the complementarity determining region CDR, the weight in a case where the distance from the centroid is less than the threshold value is 2, and the weight in a case where the distance from the centroid is equal to or more than the threshold value is 2.5. In a case where the partition region is a region other than the complementarity determining region CDR, the weight in a case where the distance from the centroid is less than the threshold value is 1, and the weight in a case where the distance from the centroid is equal to or more than the threshold value is 1.5. In a case where the distance from the centroid is equal to or more than the threshold value, the weight is set to be higher than in a case where the distance from the centroid is less than the threshold value.

The larger the distance from the centroid is, the more the amino acid residue is generally exposed to the solvent. Therefore, in a case where the three-dimensional structural information is set as the information on the three-dimensional coordinates of each amino acid residue and the centroid coordinates of the antibody 16, and the weight of the feature amount of the amino acid residue in which the distance from the centroid is equal to or more than the threshold value is set to be higher than the weight of the feature amount of the amino acid residue in which the distance from the centroid is less than the threshold value, it is possible to predict the property information 17 with higher accuracy. In a case where the property information 17 is related to the storage solution of the antibody 16 such as the optimal hydrogen ion exponent of the antibody 16, the effect is particularly enhanced.

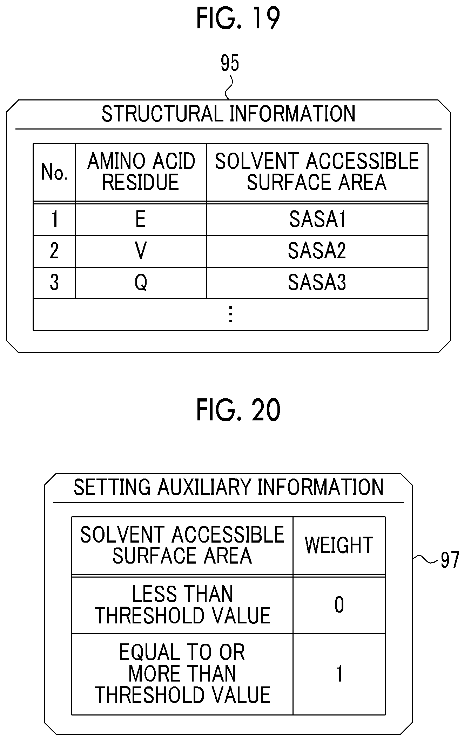

In addition, as the structural information 95 shown in FIG. 19 as an example, the three-dimensional structural information may be information on a solvent accessible surface area (SASA) of each amino acid residue. The solvent accessible surface area is derived based on the three-dimensional coordinates of each amino acid residue.

In this case, the weight setting unit 44 sets the weight by referring to setting auxiliary information 97 shown in FIG. 20 as an example. The setting auxiliary information 97 is setting auxiliary information in which the weight in a case where the solvent accessible surface area is less than the threshold value is 0, and the weight in a case where the solvent accessible surface area is equal to or more than the threshold value is 1. The feature amount of the amino acid residue in which the weight is set to 0 is not added in a case of calculating the weighted average of the preprocessing. As can be seen from this example, the weight set by the weight setting unit 44 may include 0.

The larger the solvent accessible surface area is, the more the amino acid residue is exposed to the solvent. Therefore, in a case where the three-dimensional structural information is set as the information on the solvent accessible surface area, and the weight of the feature amount of the amino acid residue in which the solvent accessible surface area is equal to or more than the threshold value is set to be higher than the weight of the feature amount of the amino acid residue in which the solvent accessible surface area is less than the threshold value, it is possible to predict the property information 17 with higher accuracy. In a case where the property information 17 is related to the storage solution of the antibody 16 such as the optimal hydrogen ion exponent of the antibody 16, the effect is particularly enhanced.

### [Second Embodiment]

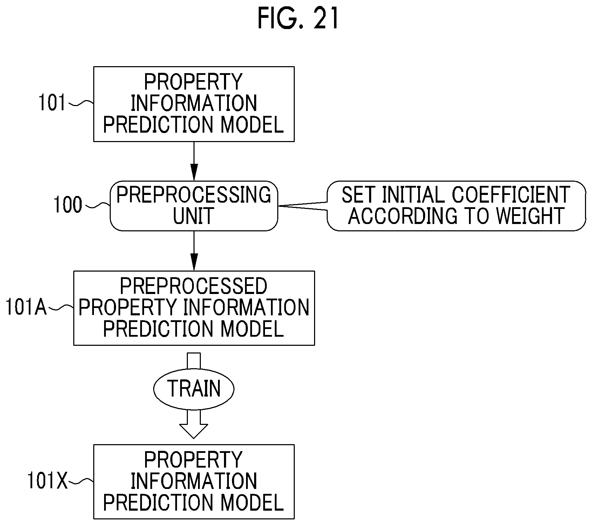

As shown in FIG. 21 as an example, a preprocessing unit 100 of the second embodiment performs preprocessing according to the weight on a property information prediction model 101 before training, thereby obtaining a preprocessed property information prediction model 101A. In the property information prediction model 38 of the first embodiment, each feature amount of the aggregated feature amount 52 is input to each node ND of the input layer 61, but in the property information prediction model 101 of the second embodiment, each feature amount of the feature amount group 51 is input to each node ND of the input layer 61.

The preprocessing unit 100 sets an initial coefficient according to the weight between each node ND of the property information prediction model 101 before training as the preprocessing. Specifically, the initial coefficient between the nodes ND related to the feature amount having a relatively high weight is set to be higher than the coefficient between the nodes ND related to the feature amount having a relatively low weight such that the feature amount having a relatively high weight is treated as being more important. By training the preprocessed property information prediction model 101A in which the initial coefficient is set, a property information prediction model 101X used in the prediction unit 46 is obtained.

As described above, in the second embodiment, the preprocessing is processing for setting the initial coefficient according to the weight in the property information prediction model 101. Therefore, the preprocessed property information prediction model 101A can be trained to converge with a small amount of training data, and the property information prediction model 101X having a relatively high prediction accuracy can be easily obtained.

The structural information may be derived by a device other than the information processing server 10, and transmitted from the device to the information processing server 10 via the network 12 or the like. Similarly, the feature amount may be extracted by a device other than the information processing server 10, and transmitted from the device to the information processing server 10 via the network 12 or the like.

As the feature amount of the amino acid residue, a spatial aggregation propensity (SAP), a spatial charge map (SCM), or the like may be adopted.

The property information 17 is not limited to the optimal hydrogen ion exponent given as an example. The viscosity of the storage solution of the antibody 16 in which the quality of the antibody 16 is most stably maintained, a type of an additive to be added to the storage solution such as L-arginine hydrochloride or purified white sugar, a concentration of the additive, or the like may be used. In addition, an indicator indicating ease of aggregation of the antibody 16, an indicator indicating hydrophobicity of the antibody 16, the presence or absence of toxicity of the antibody 16, or the like may be used.

The protein is not limited to the antibody 16 given as an example. A peptide, a nucleic acid, or the like may be used. Examples of the protein include cytokine (interferon, interleukin, or the like), hormone (insulin, glucagon, follicle-stimulating hormone, erythropoietin, or the like), a growth factor (insulin-like growth factor (IGF)-1, basic fibroblast growth factor (bFGF), or the like), a blood coagulation factor (seventh factor, eighth factor, ninth factor, or the like), an enzyme (lysosomal enzyme, deoxyribonucleic acid (DNA) degrading enzyme, or the like), a fragment crystallizable (Fc) fusion protein, a receptor, albumin, and a protein vaccine. In addition, examples of the antibody include a bispecific antibody, an antibody-drug conjugate, a low-molecular-weight antibody, a sugar-chain-modified antibody, and the like.

The property information 17 itself may be delivered from the information processing server 10 to the user terminal 11, instead of delivering the screen data of the prediction result display screen 80 including the message representing the property information 17 to the user terminal 11.

The aspect in which the property information 17 is provided for the user U to view is not limited to the prediction result display screen 80 given as an example. A printed matter of the property information 17 may be provided to the user U, or an electronic mail to which the property information 17 is attached may be transmitted to a mobile terminal of the user U.

The information processing server 10 may be installed in each pharmaceutical facility or may be installed in a data center independent of the pharmaceutical facility. In addition, the user terminal 11 may have a part or all of the functions of the respective processing units 40 to 47 of the information processing server 10.

The hardware configuration of the computer constituting the information processing server 10 according to the technology of the present disclosure can be variously modified. For example, the information processing server 10 can be configured by using a plurality of computers separated as hardware for the purpose of improving processing capacity and reliability. For example, the functions of the reception unit 40, the RW control unit 41, the structural information derivation unit 42, and the feature amount extraction unit 43, and the functions of the weight setting unit 44, the preprocessing unit 45, the prediction unit 46, and the screen delivery control unit 47 are distributed to two computers. In this case, the information processing server 10 is configured by using two computers.

As described above, the hardware configuration of the computer of the information processing server 10 can be changed as appropriate in accordance with required performance, such as processing capacity, safety, and reliability. Further, it goes without saying that, in addition to the hardware, the APs, such as the operation program 35 and the prediction AP 70, can be duplicated or distributed and stored in a plurality of storages for the purpose of securing safety and reliability.

In each of the above-described embodiments, for example, the following various processors can be used as a hardware structure of a processing unit that executes various types of processing, such as the reception unit 40, the RW control unit 41, the structural information derivation unit 42, the feature amount extraction unit 43, the weight setting unit 44, the preprocessing units 45 and 100, the prediction unit 46, the screen delivery control unit 47, and the browser control unit 72. The various processors include, for example, the CPUs 27A and 27B which are general-purpose processors executing software (the operation program 35 and the prediction AP 70) to function as various processing units as described above, a programmable logic device (PLD), such as a field programmable gate array (FPGA), which is a processor whose circuit configuration can be changed after manufacture, and a dedicated electric circuit, such as an application specific integrated circuit (ASIC), which is a processor having a dedicated circuit configuration designed to perform a specific process.

One processing unit may be configured by one of the various types of processors or may be configured by a combination of two or more processors of the same type or different types (for example, a combination of a plurality of FPGAs and/or a combination of a CPU and an FPGA). Further, a plurality of processing units may be configured by one processor.

As an example of configuring the plurality of processing units with one processor, first, there is a form in which one processor is configured by a combination of one or more CPUs and software, as typified by computers such as a client and a server, and the processor functions as the plurality of processing units. Second, as represented by a system on chip (SoC) or the like, there is a form in which a processor, which implements the functions of the entire system including the plurality of processing units with a single integrated circuit (IC) chip, is used. As described above, the various processing units are configured by using one or more of the above various processors as the hardware structure.

In addition, more specifically, an electric circuit (circuitry) in which circuit elements, such as semiconductor elements, are combined can be used as the hardware structure of the various processors.

It is possible to understand the technology described in the following supplementary notes from the above description.

### [Supplementary Note 1]

An information processing apparatus comprising:
a processor,
wherein the processor is configured to:
   acquire structural information of an amino acid sequence unique to a protein and a feature amount of an amino acid residue constituting the amino acid sequence;
   set a weight of the feature amount based on the structural information; and
   cause a machine learning model to predict property information indicating a property of the protein by applying the feature amount to which the weight is set to the machine learning model.

### [Supplementary Note 2]

The information processing apparatus according to Supplementary Note 1,
wherein the structural information is partition region information indicating a functional partition region of the amino acid residue.

### [Supplementary Note 3]

The information processing apparatus according to Supplementary Note 2,
wherein the protein is an antibody,
the partition region includes a complementarity determining region, and
the processor is configured to set a weight of a feature amount of the amino acid residue in which the partition region indicated by the partition region information is the complementarity determining region to be higher than a weight of a feature amount of the amino acid residue in which the partition region indicated by the partition region information is not the complementarity determining region.

### [Supplementary Note 4]

The information processing apparatus according to any one of Supplementary Notes 1 to 3,
wherein the structural information is three-dimensional structural information of the amino acid sequence.

### [Supplementary Note 5]

The information processing apparatus according to Supplementary Note 4,
wherein the three-dimensional structural information is three-dimensional shape information indicating a three-dimensional shape of the amino acid sequence.

### [Supplementary Note 6]

The information processing apparatus according to Supplementary Note 5,
wherein the three-dimensional shape includes a loop shape and a linear shape, and
the processor is configured to set a weight of a feature amount of the amino acid residue in which the three-dimensional shape indicated by the three-dimensional shape information is the loop shape to be higher than a weight of a feature amount of the amino acid residue in which the three-dimensional shape indicated by the three-dimensional shape information is the linear shape.

### [Supplementary Note 7]

The information processing apparatus according to any one of Supplementary Notes 1 to 6,
wherein the processor is configured to perform preprocessing according to the weight on the feature amount of the amino acid residue or the machine learning model.

### [Supplementary Note 8]

The information processing apparatus according to Supplementary Note 7,
wherein the preprocessing is processing for calculating a weighted average of the feature amounts of the amino acid residues according to the weight.

### [Supplementary Note 9]

The information processing apparatus according to Supplementary Note 7,
wherein the preprocessing is processing for setting an initial coefficient according to the weight in the machine learning model.

### [Supplementary Note 10]

The information processing apparatus according to any one of Supplementary Notes 1 to 9,
wherein the property information is an optimal hydrogen ion exponent of the protein.

### [Supplementary Note 11]

The information processing apparatus according to any one of Supplementary Notes 1 to 10,
wherein the protein is an antibody.

The above various embodiments and/or various modification examples can be combined as appropriate in the technology of the present disclosure. In addition, it is needless to say that the present disclosure is not limited to each of the above-described embodiments, and various configurations can be used without departing from the gist of the present disclosure. Furthermore, the technology of the present disclosure extends to a storage medium that non-transitorily stores the program, and a computer program product including the program, in addition to the program.

The description content and the illustrated content described above are detailed descriptions of portions according to the technology of the present disclosure and are merely an example of the technology of the present disclosure. For example, the above description of the configuration, the function, the operation, and the effect are the description of examples of the configuration, the function, the operation, and the effect of the parts according to the technology of the present disclosure. Accordingly, it goes without saying that unnecessary parts may be deleted, new elements may be added, or replacements may be made with respect to the above-described contents and the above-shown contents within a range that does not deviate from the gist of the technology of the present disclosure. In order to avoid complication and facilitate understanding of the portion according to the technology of the present disclosure, the description related to common general knowledge not requiring special description in order to implement the technology of the present disclosure is omitted in the above description content and illustrated content.

In the present specification, "A and/or B" is synonymous with "at least one of A or B". That is, "A and/or B" means that A alone may be used, B alone may be used, or a combination of A and B may be used.

Further, in the present specification, in a case where three or more items are expressed in combination using "and/or", the same concept as that of "A and/or B" applies.

All documents, patent applications, and technical standards described in the present specification are incorporated in the present specification by reference to the same extent as in a case where each of the documents, patent applications, and technical standards are specifically and individually indicated to be incorporated by reference.

## Claims

1. An information processing apparatus comprising:
a processor,
wherein the processor is configured to:
acquire structural information of an amino acid sequence unique to a protein and a feature amount of an amino acid residue constituting the amino acid sequence;
set a weight of the feature amount based on the structural information; and
cause a machine learning model to predict property information indicating a property of the protein by applying the feature amount to which the weight is set to the machine learning model.

2. The information processing apparatus according to claim 1,
wherein the structural information is partition region information indicating a functional partition region of the amino acid residue.

3. The information processing apparatus according to claim 2,
wherein the protein is an antibody,
the partition region includes a complementarity determining region, and
the processor is configured to set a weight of a feature amount of the amino acid residue in which the partition region indicated by the partition region information is the complementarity determining region to be higher than a weight of a feature amount of the amino acid residue in which the partition region indicated by the partition region information is not the complementarity determining region.

4. The information processing apparatus according to claim 1,
wherein the structural information is three-dimensional structural information of the amino acid sequence.

5. The information processing apparatus according to claim 4,
wherein the three-dimensional structural information is three-dimensional shape information indicating a three-dimensional shape of the amino acid sequence.

6. The information processing apparatus according to claim 5,
wherein the three-dimensional shape includes a loop shape and a linear shape, and
the processor is configured to set a weight of a feature amount of the amino acid residue in which the three-dimensional shape indicated by the three-dimensional shape information is the loop shape to be higher than a weight of a feature amount of the amino acid residue in which the three-dimensional shape indicated by the three-dimensional shape information is the linear shape.

7. The information processing apparatus according to claim 1,
wherein the processor is configured to perform preprocessing according to the weight on the feature amount of the amino acid residue or the machine learning model.

8. The information processing apparatus according to claim 7,
wherein the preprocessing is processing for calculating a weighted average of the feature amounts of the amino acid residues according to the weight.

9. The information processing apparatus according to claim 7,
wherein the preprocessing is processing for setting an initial coefficient according to the weight in the machine learning model.

10. The information processing apparatus according to claim 1,
wherein the property information is an optimal hydrogen ion exponent of the protein.

11. The information processing apparatus according to claim 1,
wherein the protein is an antibody.

12. An operation method of an information processing apparatus, the operation method comprising:
acquiring structural information of an amino acid sequence unique to a protein and a feature amount of an amino acid residue constituting the amino acid sequence;
setting a weight of the feature amount based on the structural information; and
causing a machine learning model to predict property information indicating a property of the protein by applying the feature amount to which the weight is set to the machine learning model.

13. An operation program of an information processing apparatus, the operation program causing a computer to execute a process comprising:
acquiring structural information of an amino acid sequence unique to a protein and a feature amount of an amino acid residue constituting the amino acid sequence;
setting a weight of the feature amount based on the structural information; and
causing a machine learning model to predict property information indicating a property of the protein by applying the feature amount to which the weight is set to the machine learning model.
